# EUROPEAN PATENT SPECIFICATION

(11) **EP 0 637 453 B1**
(45) Date of publication and mention of the grant of the patent: **12.01.2000**
(21) Application number: 94201655.1
(22) Date of filing: 09.06.1994
(51) Int. Cl.: A61M 25/00

(54) **Method for manufacturing a catheter with at least one high-pressure lumen and catheter**
Verfahren zur Herstellung eines Katheters mit mindestens einem Hochdrucklumen und Katheter
Procédé de fabrication d'un cathéter avec au moins un tube haute-pression et cathéter

(30) Priority: 05.07.1993 NL 9301181
(43) Date of publication of application: 08.02.1995
(73) Proprietor: CORDIS EUROPA N.V., NL-9301 LJ Roden (NL)
(72) Inventor: Boudewijn, Alexander Christiaan, NL-9351 KS Leek (NL); Noppert, Tiemen, NL-8427 RK Ravenswoud (NL)
(74) Representative: 't Jong, Bastiaan Jacobus

(56) References cited:
- DE-A- 3 239 032
- FR-A- 2 530 958
- US-A- 4 100 246

## Description

The invention relates to a hydro-thrombectomy catheter and a method for manufacturing such a catheter with at least one high-pressure lumen wherein, although the cross-section of this lumen is small, a considerable liquid flow can be generated, and one low-pressure lumen at a distal end connected with an opening in the catheter through which blood clots can be removed, comprising the arranging of connecting members at one end forming connections with the lumens.

Such a catheter is for example known from EP-A-0 442 579 from the applicant. The hydro-thrombectomy catheter described therein comprises a high-pressure lumen which bends back at the distal end and ends in a nozzle which is directed along an opening in the low-pressure lumen. By supplying a liquid under high pressure through the high-pressure lumen close to the opening in the low-pressure lumen, a suction is created by ejector action with which for example thrombi can be removed. This known hydro-thrombectomy catheter is made from a single tube comprising both the low pressure lumen and the high pressure lumen.

The invention is distinguished by a thin tube-like element with a high-pressure resistant sheath defining the high-pressure lumen, the incorporation of the thin tube-like element in a thicker tube-like element whilst leaving space for a longitudinal channel to form the low-pressure lumen, said thin tube-like element extending over substantially the same length of the catheter as the thicker tube-like element and the method of manufacturing the catheter. By using a separate, thin tube-like element with a high-pressure resistant sheath which defines the high-pressure lumen only, the catheter can withstand very high pressures in the high-pressure lumen and suction efficiency thereof can safely be enhanced. The thicker tube-like element in which the thin tube-like element has been incorporated can be manufactured of a different material, for instance a far less pressure-resistant and more pliable material. The manufactured catheter will display the required flexibility, notwithstanding the rigidity corresponding to high pressure resistance for the high-pressure lumen. The influence of more rigid, highly pressure-resistant material for the high-pressure lumen only is considerably lower than if both lumens were to be manufactured from such material. Moreover, said influence can reliably and favourably be compensated by the choice for a material for the low-pressure lumen with a higher flexibility corresponding with the lower pressure resistance requirements for the low-pressure lumen.

An advantageous embodiment of the method is characterised in claim 2. The thin tube-like element is used as mandrel around which the thicker tube-like element is extruded.

Another embodiment of the method according to the invention is characterised in claim 3. Incorporating the thin tube-like element in the thicker one occurs during the final stage of manufacturing the catheter, whereby the tube-like elements to be joined have a length matching the length of the catheter to be manufactured.

When a strong adhesion of the thin tube-like element in the thicker one is required, the measure as set out in claim 4 is preferably employed. The thin tube-like element has in that case its own space in which it has been incorporated in the thicker tube-like element.

A simple embodiment of the method which can be carried out very easily, is characterised in claim 5. The low-pressure lumen in the thicker tube-like element can thus be given the maximum effective cross-section. The thin tube-like element can very easily be incorporated in the thicker one, as it is surrounded by space in the channel forming the low-pressure lumen.

In order to obtain the required compression resistance of the thin tube-like element, the measure as set out in claim 6 is preferably employed. Reinforcement of the plastic material can be achieved by employing a known extrusion-method wherein during the extrusion 'threads' of a plastic material, which in solidified state have a high tensile strength, are extruded simultaneously in contrastive helically shaped bands in the extrusion profile.

Another possibility is characterised in claim 7. With the present standard of technology, very thin tube-like elements with a very thin wall can be manufactured in this way which, due to the reinforcing layer, have a very high compression resistance.

The invention relates to and also provides a catheter as characterised in claims 8-16.

The invention will be explained in greater detail in the following description with reference to the attached drawings.
Fig. 1 shows schematically part of a catheter manufactured by the method of the invention.
Fig. 2 illustrates schematically a manufacturing method of the invention.
Fig. 3 shows, partly in a longitudinal cross-section, part of a catheter manufactured by the method of the invention.
Fig. 4 shows a cross-section of fig. 3.
Fig. 5 and 6 represent views of a catheter manufactured by another method of the invention corresponding to fig. 3.
Fig. 7 shows a cross-section of a catheter manufactured by yet another method of the invention corresponding to fig. 4.

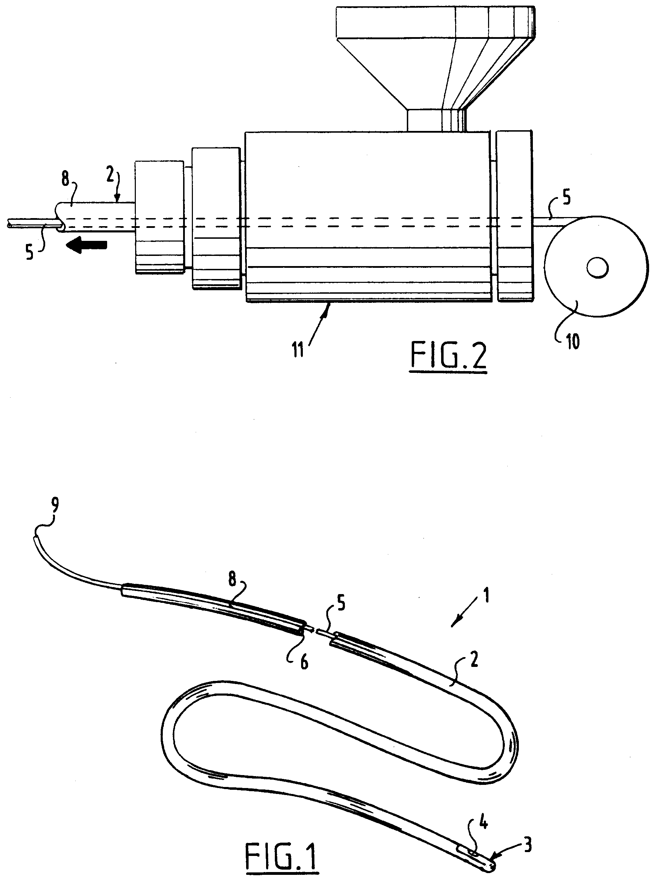

The catheter 1, partly shown in fig. 1, comprises in the usual manner a basic body 2 with a distal end 3 which is introduced into the body of a patient during treatment. The proximal end, to which the connecting pieces have been attached, is not shown in fig. 1.

The catheter 1 is of the thrombectomy type and has an opening 4 in the distal end 3 through which for example blood clots can be removed from the body of a patient. With this catheter the suction is generated by an ejector action at the opening 4, which is achieved as a liquid jet inside the catheter is directed along the opening 4.

The liquid for this liquid jet is supplied through a high-pressure lumen which bends back at the distal end 3 of the catheter 1 and ends in a nozzle, which is directed along this opening 4.

With the catheter 1, which has been manufactured by the method of the invention, the high-pressure lumen 9 has been formed in a separate thin tube-like element 5 which has been incorporated in a thicker tube-like element 2 in which also a low-pressure lumen has been formed which is connected to the opening 4. The low-pressure lumen 6 and the high-pressure lumen 9 are connected to the connecting pieces at the proximal end of the catheter 1 mentioned above, in the usual manner. The thin tube-like element 5 has a high-pressure resistant sheath, so that a high pressure can be generated in the high-pressure lumen, wherein, although the cross-section of this lumen is small, a considerable liquid flow can be generated.

Fig. 2 shows schematically the method according to an embodiment of the invention.

A great length of the thin tube-like element 5 has been manufactured beforehand by means of an extrusion process for instance. The material used is such that the required high-pressure resistance of the thin tube-like element 5 is obtained.

The manufactured thin tube-like element 5 is wound on a storage reel 10 and is fed through a schematically illustrated extrusion machine 11. The thin tube-like body functions as mandrel during the extrusion of the thick tube-like body 8 around it. The thin tube-like element 5 is incorporated in the thicker tube-like element 8, as this thicker tube-like element is extruded around the thin one.

Thus a great length of basic material can be manufactured comprising a thicker tube-like element 8 with incorporated in it thin tube-like element 5.

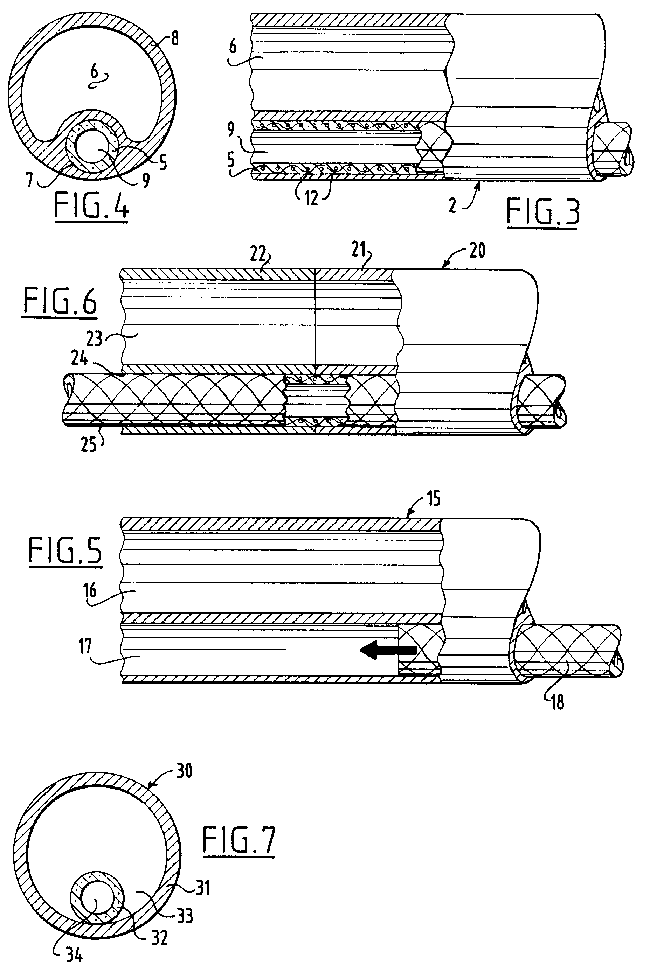

Fig. 3 shows the basic material made in this way in greater detail.

The sheath of the thin tube-like element 5 consists of reinforced plastic material. More in particular, this element 5 has been made up of an inner basic layer of a suitable plastic material, a reinforcing layer of thin metal wire 12 braided or wound around it, and surrounding it all an outer layer of plastic material. This method for manufacturing a pressure resistant tube-like element is in itself known for making catheters for instance.

The thicker tube-like element 8 in which the thin one 5 has been incorporated, is formed in such a way during the extrusion process that two channels or lumens are formed. The first lumen 7 will contain the thin pressure resistant element 5 and the other lumen will form the low-pressure lumen 6.

Fig. 5 illustrates another method. Hereby the thicker tube-like element 15 is extruded separately with two channels 16 and 17. The channel 16 forms the low-pressure lumen and the channel 17 will contain the thin tube 18 with the high-pressure lumen. During the manufacturing process suitable lengths of the thin tube 18 and the thicker tube 15 are taken from a supply of it and the thin tube-like element 18 is pushed into the channel 17 made for this purpose. In order to be able to push the tube-like element 18 properly into the channel 17, the thicker tube-like element 15 can be compressed, for instance by pushing it together on a mandrel inserted in channel 16. Consequently the diameter of the channel 17 will increase slightly. After inserting the thin tube 18, the thicker tube-like element 15 is straightened out again as a result of which the thin tube 18 will be stuck tightly inside the channel 17. A lubricant can of course be used in order to facilitate this process.

The catheter 20 shown in fig. 6 has been manufactured in this way.

The thicker tube-like element is however made up of a number of aligned parts 21 and 22. The parts 21 and 22 have different material properties so that for instance the constituent parts become gradually more pliable towards the distal end.

Each of the parts 21 and 22 has a cross-section corresponding to the cross-section of the thicker tube-like part 8, as shown in fig. 4. The channel 23 with the largest bore forms in this case once again the low-pressure lumen and the channel 24 is designed to house the thin, high-pressure tube 25. The different parts 21, 22 are placed against each other end to end and connected for instance by glueing or welding.

Although embodiments have been described here comprising one high-pressure lumen and one low-pressure lumen other configurations are possible as well of course. A catheter may comprise several high-pressure lumens and also several low-pressure lumens. The illustrated embodiments are however, for the described application as hydro-thrombectomy catheter, the appropriate embodiment.

With the embodiment of fig. 7, the thin tube-like element 32 with the high-pressure resistant sheath, has been incorporated in a thicker tube 31 which defines only one channel. That part of the cross-section not occupied by the thin tube 32 forms in that case the low-pressure lumen.

It is obvious that this catheter 30, as shown in fig. 7, can be manufactured simply by pushing the thin tube 32 into the channel of the thicker tube 31. The cross-section of the remaining low-pressure lumen 33 has in this embodiment the maximum dimension as no additional material is present for housing the thin tube-like element 32 which defines the high-pressure lumen 34. The thicker tube-like element 31 of this embodiment can be manufactured cheaply so that the entire catheter manufactured in this way also entails few costs.

As mentioned before, the thicker tube-like element can be manufactured of a relatively flexible material as this material itself is not subjected to high pressures. Thus the catheter manufactured according to the method of the invention can display the required flexibility especially at its distal end.

## Claims

1. Method for manufacturing a hydro-thrombectomy catheter (1) with at least one high-pressure lumen (9) wherein, although the cross-section of this lumen is small, a considerable liquid flow can be generated, and one low-pressure lumen (6) at a distal end (3) connected with an opening (4) in the catheter through which blood clots can be removed, comprising the arranging of connecting members at one end forming connections with the lumens, characterized by the manufacturing of a thin tube-like element (5) with a high-pressure resistant sheath defining the high-pressure lumen (9), the incorporation of the thin tube-like element (5) in a thicker tube-like element (2) whilst leaving space for a longitudinal channel to form the low-pressure lumen (6), said thin tube-like element (5) extending over substantially the same length of the catheter as the thicker tube-like element (2).

2. Method according to claim 1, wherein the thicker tube-like element is extruded around the thin one.

3. Method according to claim 1, wherein the thin tube-like element is pushed inside a longitudinal channel of the thicker tube-like element.

4. Method according to claim 3, wherein the thicker tube-like element is an extrusion profile with two lumens in one of which the thin tube-like element has been incorporated and wherein the other forms the low-pressure lumen.

5. Method according to claim 3, wherein the thin tube-like element is incorporated in a longitudinal channel of the thicker element which has a larger internal diameter than the external diameter of the thin element.

6. Method according to one of the previous claims, wherein the thin tube-like element consists of a reinforced plastic material.

7. Method according to claim 6, wherein the thin tube-like element is made up of an internal basic layer of plastic material, a reinforcing layer of thin metal wire braided or wound around it and an outer layer of plastic material.

8. Hydro-thrombectomy catheter (1) comprising a catheter body (2) having at least one high-pressure lumen (9) wherein, although the cross-section of this lumen is small, a considerable liquid flow can be generated, and one low-pressure lumen (6) at a distal end (3) connected with an opening (4) in the catheter through which blood clots can be removed, wherein at a proximal end connecting members have been arranged forming connections with the lumens, characterized by a thin tube-like element (5) with a high-pressure resistant sheath defining the high-pressure lumen (9), which has been incorporated in a thicker tube-like element (2) whilst leaving space for a longitudinal channel forming the low-pressure lumen (6) and said thin tube-like element (5) extending over substantially the same length of the catheter as the thicker tube-like element (2).

9. Catheter as claimed in claim 8, obtainable by extrusion of the thicker tube-like element around the thin one.

10. Catheter as claimed in claim 8, obtainable by pushing of the thin tube-like element inside a longitudinal channel of the thicker tube-like element.

11. Catheter as claimed in claim 10, obtainable by provision of the thicker tube-like element as an extrusion profile with two lumens in one of which the thin tube-like element has been incorporated and wherein the other one forms the low-pressure lumen.

12. Catheter as claimed in claim 10, obtainable by incorporation of the thin tube-like element in a longitudinal channel of the thicker element which has a larger internal diameter than the external diameter of the thin element.

13. Catheter as claimed in one of the claims 8 - 11, wherein the thin tube-like element consists of reinforced plastic material.

14. Catheter as claimed in claim 13, wherein the thin tube-like element is made up of an internal basic layer of plastic material, a reinforcing layer of thin metal wire braided or wound around it and an outer layer of plastic material.

15. Catheter as claimed in one of the claims 8 - 14, wherein the thicker tube-like element is made of a more pliable material than the thin tube-like element.

16. Catheter as claimed in claim 15, wherein the thicker tube-like element consists of a number of aligned parts with different properties.

## Patentansprüche

1. Verfahren zur Herstellung eines Hydrothrombektomie-Katheters (1) mit mindestens einem Hochdrucklumen (9), in welchem, obwohl der Querschnitt dieses Lumens klein ist, ein beträchtlicher Flüssigkeitsfluß erzeugt werden kann, und einem Niederdrucklumen (6), das an einem distalen Ende (3) mit einer Öffnung (4) in dem Katheter verbunden ist, durch die Blutpfröpfe entfernt werden können, umfassend die Anordnung von Verbindungselementen an einem Ende, die Verbindungen mit den Lumina bilden, **gekennzeichnet** durch die Herstellung eines dünnen rohrförmigen Elementes (5) mit einer hochdruckfesten Ummantelung, welches das Hochdrucklumen (9) begrenzt, das Einführen des dünnen rohrförmigen Elementes (5) in ein dickeres rohrförmiges Element (2) unter Freilassung eines Raumes für einen Längskanal, der das Niederdrucklumen (6) bildet, wobei sich das dünne rohrförmige Element (5) im wesentlichen über die gleiche Länge des Katheters wie das dickere rohrförmige Element (2) erstreckt.

2. Verfahren nach Anspruch 1, bei dem das dickere rohrförmige Element um das dünne herum extrudiert wird.

3. Verfahren nach Anspruch 1, bei dem das dünne rohrförmige Element in einen Längskanal des dickeren rohrförmigen Elementes eingeschoben wird.

4. Verfahren nach Anspruch 3, bei dem das dickere rohrförmige Element ein Extrusionsprofil mit zwei Lumina ist, in deren einem das dünne rohrförmige Element eingelagert worden ist und deren anderes das Niederdrucklumen bildet.

5. Verfahren nach Anspruch 3, bei dem das dünne rohrförmige Element in einem Längskanal des dickeren Elements eingelagert ist, der einen größeren Innendurchmesser als der Außendurchmesser des dünnen Elements aufweist.

6. Verfahren nach einem der vorangehenden Ansprüchen, bei dem das dünne rohrförmige Element aus einem verstärkten Kunststoffmaterial besteht.

7. Verfahren nach Anspruch 6, bei dem das dünne rohrförmige Element aus einer inneren Grundschicht aus Kunststoffmaterial, einer Verstärkungsschicht aus darum geflochtenem oder gewickeltem dünnen Metalldraht, und einer Außenschicht aus Kunststoffmaterial besteht.

8. Hydrothrombektomie-Katheter (1) mit einem Katheterkörper (2) mit mindestens einem Hochdrucklumen (9), in welchem, obwohl der Querschnitt dieses Lumens klein ist, ein beträchtlicher Flüssigkeitsfluß erzeugt werden kann, und mindestens einem Niederdrucklumen (6), das an einem distalen Ende (3) mit einer Öffnung (4) in dem Katheter verbunden ist, durch die Blutpfropfen entfernt werden können, wobei an einem proximalen Ende Verbindungselemente angeordnet sind, die Verbindungen mit den Lumina bilden, **gekennzeichnet** durch ein dünnes rohrförmiges Element (5) mit einer hochdruckfesten Ummantelung, welches das Hochdrucklumen (9) begrenzt, welches in ein dickeres rohrförmiges Element (2) eingelagert worden ist unter Freilassung eines Raumes für einen Längskanal, der das Niederdrucklumen (6) bildet, und daß das dünne rohrförmige Element (5) sich über im wesentlichen die gleiche Länge des Katheters erstreckt wie das dickere rohrförmige Element (2).

9. Katheter nach Anspruch 8, erhältlich durch Extrudieren des dickeren rohrförmigen Elementes um das dünnere herum.

10. Katheter nach Anspruch 8, erhältlich durch Einschieben des dünneren rohrförmigen Elementes in einen Längskanal des dickeren rohrförmigen Elementes.

11. Katheter nach Anspruch 10, erhältlich durch Herstellung des dickeren rohrförmigen Elementes als ein Extrusionsprofil mit zwei Lumina, in deren einem das dünne rohrförmige Element eingelagert worden ist und deren anderes das Niederdrucklumen bildet.

12. Katheter nach Anspruch 10, erhältlich durch Einlagern des dünnen rohrförmigen Elementes in einen Längskanal des dickeren Elementes, der einen größeren Innendurchmesser als der Außendurchmesser des dünnen Elementes hat.

13. Katheter nach einem der Ansprüche 8 bis 11, bei dem das dünne rohrförmige Element aus verstärktem Kunststoffmaterial besteht.

14. Katheter nach Anspruch 13, bei dem das dünne rohrförmige Element aus einer inneren Grundschicht aus Kunststoffmaterial, einer verstärkten Schicht aus darum geflochtenem oder gewickeltem dünnen Metalldraht, und einer Außenschicht aus Kunststoffmaterial besteht.

15. Katheter nach einem der Ansprüche 8 bis 14, bei dem das dickere rohrförmige Element aus einem flexibleren Material als das dünne rohrförmige Element besteht.

16. Katheter nach Anspruch 15, bei dem das dickere rohrförmige Element aus einer Anzahl von aneinandergereihten Teilen mit unterschiedlichen Eigenschaften besteht.

## Revendications

1. Procédé de fabrication d'un cathéter pour hydro-thrombectomie (1) comprenant au moins un tube haute pression (9) dans lequel, bien que la section transversale de ce tube soit petite, un écoulement important de liquide peut être généré, et un tube basse pression (6) raccordé à une extrémité distale (3) à une ouverture (4) ménagée dans le cathéter à travers laquelle les caillots sanguins peuvent être éliminés, comprenant l'agencement d'éléments de raccordement à une extrémité formant des raccords avec les tubes, caractérisé par la fabrication d'un élément tubulaire mince (5) muni d'une gaine résistant aux hautes pressions définissant le tube haute pression (9), l'incorporation de l'élément tubulaire mince (5) dans un élément tubulaire plus épais (2) tout en laissant un espace pour un canal longitudinal pour qu'il forme le tube basse pression (6), ledit élément tubulaire (5) s'étendant sur sensiblement la même longueur du cathéter que l'élément tubulaire plus épais (2).

2. Procédé selon la revendication 1, dans lequel l'élément tubulaire plus épais est extrudé autour de l'élément tubulaire mince.

3. Procédé selon la revendication 1, dans lequel l'élément tubulaire mince est poussé à l'intérieur d'un canal longitudinal de l'élément tubulaire plus épais.

4. Procédé selon la revendication 3, dans lequel l'élément tubulaire plus épais est un profil d'extrusion avec deux tubes dans l'un desquels est incorporé l'élément tubulaire mince et dans lequel l'autre forme le tube basse pression.

5. Procédé selon la revendication 3, dans lequel l'élément tubulaire mince est incorporé dans un canal longitudinal de l'élément plus épais qui présente un diamètre intérieur plus important que le diamètre extérieur de l'élément mince.

6. Procédé selon l'une des revendications précédentes, dans lequel l'élément tubulaire mince se compose d'une matière plastique renforcée.

7. Procédé selon la revendication 6, dans lequel l'élément tubulaire mince est fabriqué à l'aide d'une couche de base intérieure en matière plastique, d'une couche de renfort en fil métallique mince tressé ou enroulé autour de celle-ci et d'une couche extérieure en matière plastique.

8. Cathéter d'hydro-thrombectomie (1) comprenant un corps de cathéter (2) ayant au moins un tube haute pression (9) dans lequel, bien que la section transversale de ce tube soit petite, un écoulement important de liquide peut être généré, et un tube basse pression (6) raccordé à une extrémité distale (3) à une ouverture (4) ménagée dans le cathéter, à travers laquelle les caillots sanguins peuvent être éliminés, dans lequel, à une extrémité proximale, des éléments de raccordement ont été agencés pour former des raccords avec les tubes, caractérisé par un élément tubulaire mince (5) muni d'une gaine résistant aux hautes pressions définissant le tube haute pression (9), qui est incorporé dans un élément tubulaire plus épais (2) tout en laissant un espace pour un canal longitudinal formant le tube basse pression (6) et ledit élément tubulaire mince (5) s'étendant sur sensiblement la même longueur du cathéter que l'élément tubulaire plus épais (2).

9. Cathéter selon la revendication 8, pouvant être obtenu en extrudant l'élément tubulaire plus épais autour de l'élément tubulaire mince.

10. Cathéter selon la revendication 8, pouvant être obtenu en poussant l'élément tubulaire mince à l'intérieur d'un canal longitudinal de l'élément tubulaire plus épais.

11. Cathéter selon la revendication 10, pouvant être obtenu en prévoyant l'élément tubulaire plus épais sous forme d'un profil d'extrusion avec deux tubes dans l'un desquels l'élément tubulaire mince est incorporé et dans lequel l'autre forme le tube basse pression.

12. Cathéter selon la revendication 10, l'élément tubulaire mince pouvant être obtenu en incorporant l'élément tubulaire mince dans un canal longitudinal de l'élément plus épais qui présente un diamètre intérieur plus important que le diamètre extérieur de l'élément mince.

13. Cathéter selon l'une des revendications 8 à 11, dans lequel l'élément tubulaire mince se compose d'une matière plastique renforcée.

14. Cathéter selon la revendication 13, dans lequel l'élément tubulaire mince est fabriqué à partir d'une couche de base intérieure en matière plastique, d'une couche de renfort en fil métallique mince tressé ou enroulé autour de celle-ci et une couche extérieure en matière plastique.

15. Cathéter selon l'une des revendications 8 à 14, dans lequel l'élément tubulaire plus épais est fabriqué à partir d'une matière plus pliable que l'élément tubulaire mince.

16. Cathéter selon la revendication 15, dans lequel l'élément tubulaire plus épais se compose d'un certain nombre de pièces alignées ayant des propriétés différentes.
